# EUROPÄISCHE PATENTSCHRIFT

(11) **EP 0 627 983 B1**
(45) Veröffentlichungstag und Bekanntmachung des Hinweises auf die Patenterteilung: **09.10.1996**
(21) Anmeldenummer: 93903964.0
(22) Anmeldetag: 12.02.1993
(51) Int. Cl.: B29C 67/00

(54) **VERFAHREN ZUR HERSTELLUNG VON FORMKÖRPERN MIT VORBESTIMMTER PORENSTRUKTUR**
PROCESS FOR PRODUCING MOULDED BODIES WITH A PREDETERMINED PORE STRUCTURE
PROCEDE DE FABRICATION DE CORPS MOULES POREUX AYANT UNE STRUCTURE DE PORES DONNEE

(30) Priorität: 27.02.1992 DE 4205969
(43) Veröffentlichungstag der Anmeldung: 14.12.1994
(73) Patentinhaber: MERCK PATENT GmbH, D-64271 Darmstadt (DE); BAUER, Andrea, D-55234 Flomborn (DE)
(72) Erfinder: BAUER, Jörg, D-6509 Flomborn (DE); BAUER, Andrea, D-6509 Flomborn (DE)
(86) Internationale Anmeldenummer: EP9300344
(87) Internationale Veröffentlichungsnummer: WO9316865

(56) Entgegenhaltungen:
- EP-A- 0 253 506
- GB-A- 2 233 928
- PATENT ABSTRACTS OF JAPAN 6. Oktober 1989 & JP-A-11 73 691 (MATSUSHITA ELECTRIC IND CO LTD) 10. Juli 1989
- TECHNISCHE RUNDSCHAU Bd. 83, Nr. 20, 17. Mai 1991, BERN, CH Seiten 36 - 43 SCHÄTTI 'rapid prototyping etabliert sich'
- SCH[TTI 'rapid prototyping etabliert sich'

## Beschreibung

Die Erfindung betrifft ein Verfahren zur Herstellung von Formkörpern mit vorbestimmter Porenstruktur. Dieses Verfahren kann vorzugsweise zur Herstellung von Formkörpern mit dreidimensional interkonnektierendem Porensystem und insbesondere zur Herstellung von Implantatformkörpern mit dem Porensystem von natürlichen Knochen verwendet werden.

Die Herstellung von Formkörpern, gleich aus welchem Material und Werkstoff, mit vorbestimmter Porenstruktur ist naturgemäß problematisch. Dies ist vor allem dann der Fall, wenn statt einer material- und verarbeitungsbedingten statistisch gleichmäßigen Porosität oder einer eindimensionalen Porenausrichtung eine lokal unterschiedliche Strukturierung und/oder ein definiertes Porensystem in mehreren Dimensionen gewünscht wird. So führen bekannte Techniken zur Herstellung von porösen Materialen bzw. Körpern, wie etwa das Aufschäumen geeigneter Materialien oder die Einarbeitung von löslichen oder gasbildenden Stoffen in entsprechende Matrixmaterialien, zu im wesentlichen statistisch gleichmäßigen Porenstrukturen. Bei Extrusionsvorgängen von plastischen oder plastifizierbaren Materialien lassen sich allenfalls im wesentlichen eindimensional ausgerichtete Porensysteme erzeugen.

Diese allgemeingültige Problematik soll nun am Beispiel der Implantatformkörper für den Knochenersatz und der hier maßgebenden Erfordernisse näher erläutert werden.

Knochenersatzwerkstoffe für die Herstellung von Implantatformkörpern lassen sich in die beiden Hauptgruppen der bioinerten und der bioaktiven Materialien einteilen. Zu den bioinerten Materialien zählen physiologisch akzeptable Metalle, Oxidkeramiken und bestimmte Kunststoffe. Zu den bioaktiven Materialien, die einen wie immer gearteten positiven physiologischen Effekt im Organismus bewirken, gehören Materialien auf Basis von Kalzium-Mineralien, Biopolymere sowie Kompositwerkstoffe auf Basis der genannten Materialien. Eine Untergruppe hiervon bilden die bioresorbierbaren Materialien.

Bioaktive Implantatwerkstoffe genießen bevorzugtes Interesse, da sie eine Stimulierung des Knochenwachstums und dadurch eine verbesserte Einheilung des Implantats in den Knochen und das körpereigene Gewebe bewirken.

Formkörper aus keramischem Material werden in erster Linie für den Ersatz von tragenden Knochenstrukturen eingesetzt, die hohen mechanischen Belastungen standhalten müssen. Beispiele hierfür sind Knochenprothesen und Knochenverbindungselemente, wie etwa Markraumnägel, Knochenschrauben und Osteosyntheseplatten.

Knochenersatzmaterial auf Basis von Kalziumphosphat-Keramiken gelten aufgrund ihrer chemischen Verwandtschaft mit der Mineralphase natürlicher Knochen als bioaktiv. Natürlicher Knochen besteht in seiner Mineralphase überwiegend aus der Kalziumphosphatverbindung Hydroxylapatit. Hydroxylapatit synthetischen oder organischen Ursprungs, etwa aus natürlichem Knochenmaterial, ist daher ein häufig verwendeter Rohstoff zur Herstellung von Implantaten für den Knochenersatz.

Die Festigkeit der Verwachsung von kompakter Kalziumphosphat-Keramik mit vorhandenem Knochen ist erfahrungsgemäß überwiegend nicht befriedigend. Ein günstigeres Einwachsverhalten zeigen poröse Kalziumphosphat-Keramiken.

Die gegenwärtig verfügbaren keramischen Implantatmaterialien auf Basis von Kalziumphosphat gliedern sich in zwei grundsätzliche Gruppen.

Die erste Gruppe verwendet synthetisch hergestellte Kalziumphosphate, die zu kompakten oder porösen Körpern geformt und anschließend zur Keramik gesintert werden. Der Vorteil dieser Materialien liegt naturgemäß in der Tatsache, daß der synthetische Aufbau problemlos gezielte chemische Zusammensetzungen mit großer Exaktheit und Reproduzierbarkeit ermöglicht. Die Standardisierbarkeit der Zusammensetzung ist für medizinische Anwendungen unerläßlich.

Ein entscheidender Nachteil synthetischer Materialien ist, daß poröse Körper nur sehr schwierig und mit hohem Aufwand hergestellt werden können. So hat es nicht an Versuchen gefehlt, poröse Formkörper aus Kalziumphosphat-Keramiken in der Weise herzustellen, daß man in eine mineralische Matrix etwa organische Fasern, Gewebe oder Geflechte oder schwammartig strukturierte Körper aus organischem Material einbettet und diese dann vor oder während der Sinterung zur Keramik ausbrennt. Ein derartiges Verfahren ist beispielsweise in EP 253 506 beschrieben. Aufgrund der strukturellen Unterschiede zu natürlichem Knochen, der aufwendigen Herstellungsweise und der nur unwesentlich verbesserten Eigenschaften in Bezug auf das Einheilungsverhalten, haben sich derartige Implantatformkörper bislang noch nicht durchsetzen können. Es ist also bis heute noch nicht möglich, mit synthetischen Materialien Formkörper mit der für natürliche Knochen charakteristischen Porosität, insbesondere etwa der offenen Porosität von Spongiosaknochen, nachzustellen. Es hat sich aber herausgestellt, daß gerade diese knochentypische Porosität für eine schnelle, feste und dauerhafte Verbindung von Implantat mit dem körpereigenen Knochen essentiell ist.

Die zweite Gruppe basiert auf biologischen Systemen, wie Knochen, Algen und Korallen, die durch verschiedene Behandlungen mineralisiert und in ein keramisches System überführt werden, wobei die Struktur des mineralischen Stützgerüstes möglichst erhalten bleiben soll. Besonders die aus Knochen gewonnenen Implantate haben eine ideale Porenstruktur, die ein Traggerüst aus Balken, Bälkchen und Stegen mit allseits interkonnektierenden Poren bei Porengrößen bis etwa 2,5 mm aufweisen und die ein der Belastungsrichtung angepaßtes Stützgerüst aufbauen. Knochenkeramikimplantate zeigen daher aufgrund ihrer ausgezeichneten Übereinstimmung mit dem Porensystem natürlichen Knochens erhebliche biologische Vorteile beim Einwachsverhalten und der Einheilung in den Organismus.

Neben diesen Vorzügen haben Knochenkeramikimplantate aber auch eine Reihe von Nachteilen.

So ist zum einen ihre Herstellung aus natürlichem Knochenmaterial äußerst zeit- und arbeitsintensiv, insbesondere dann, wenn die Mineralisierung und Sinterierung zur Keramik so schonend vorgenommen werden soll, daß praktisch keine Strukturveränderungen auftreten. Entsprechende Herstellungsverfahren sind beispielsweise beschrieben in EP 141 004 und DE-PS 37 27 606.

Zum anderen sind die Implantatmaterialien aufgrund der natürlichen Herkunft der Ausgangsmaterialien in ihrer Baugröße beschränkt, so daß nicht alle Formen und Dimensionen in Form einstückiger Knochenkeramiken realisiert werden können. Da die Implantate in ihrem Strukturaufbau sowie aufgrund der Umwandlung in ein keramisches System in Dichte, Festigkeit und Elastizität nicht den zu ersetzenden Knochen bzw. Knochenbereichen entsprechen, ist bei Belastung in Folge unzureichender Kräfteverteilung und -ableitung eine Lockerung oder sogar der Ausbruch des Implantats zu befürchten.

Nachteilig an Knochenkeramik ist weiterhin, daß das als Ausgangsmaterial eingesetzte Naturprodukt Knochen in der chemischen Zusammensetzung seiner Mineralphase erheblichen naturbedingten und nichtkontrollierbaren Schwankungen unterworfen ist. Derartige Schwankungen in der Zusammensetzung haben durchaus deutlich feststellbare Einflüsse auf die biologische Aktivität des Knochenkeramikimplantats, was sich klinisch in unterschiedlichen Einwachs- und Knochenneubildungsraten manifestiert.

Der Erfindung lag daher die Aufgabe zugrunde, ein Verfahren zur Herstellung von Implantatformkörpern für den Knochenersatz aufzufinden, mit dem diese aus synthetischen Ausgangsmaterialien ohne Beschränkungen in der Formgebung und der Dimensionierung aufgebaut und dabei in vorbestimmbarer Weise so strukturiert werden können, daß das Poren- und Stegsystem weitestgehend dem humanen Knochens entspricht.

Erfindungsgemäß wird die Aufgabe dahingehend gelöst, daß man den Implantatformkörper in wiederholter Abfolge schichtenweise durch Erzeugung entsprechend dem Porensystem bildartig strukturierter Schichten und Verfestigung derselben aus einer plastisch verformbaren und anschließend verfestigbaren Masse auf Basis von Kalziumphosphat aufbaut, wobei man die Bildstrukturen der einzelnen Schichten von als Vorlage dienenden natürlichen Knochen überträgt und den Formkörper abschließend zur Keramik sintert.

Es hat sich dabei gezeigt, daß das Verfahren weder auf das Material als solches, noch auf die Anwendung für die Herstellung von Implantatformkörpern beschrankt ist. Von der Materialseite können über Kalziumphosphat-Keramiken hinaus auch Oxidkeramiken, Glaskeramiken, nichtkeramische mineralische Werkstoffe, organische Polymermaterialien sowie im gegebenen Fall die jeweiligen Vorstufen der genannten Materialien und weiterhin auch Kompositwerkstoffe aus zwei oder mehr der vorgenannten Materialien eingesetzt werden, vorausgesetzt, sie lassen sich in Form plastisch verformbarer und anschliessend verfestigbarer Massen verarbeiten. Mit solchen Massen muß die Erzeugung von bildartig strukturierten Schichten möglich sein, wobei die anschließende Verfestigung je nach Materialauswahl, spezifischer Zusammensetzung und Verarbeitungserfordernisse durch Trocknung, chemische Härtung, Temperung oder Sinterung erfolgen kann. Mit dem Verfahren können grundsätzlich beliebige poröse Formkörper mit vorbestimmter Porenstruktur in allen erdenklichen Ausgestaltungsformen und Dimensionen, die letztendlich nur fertigungstechnisch limitiert sind, hergestellt werden. Solche Formkörper können für die unterschiedlichsten Einsatzzwecke vorgesehen sein.

Gegenstand der Erfindung ist somit ein Verfahren zur Herstellung eines porösen Formkörpers durch Formgebung einer plastisch verformbaren und anschließend verfestigbaren Masse, das dadurch gekennzeichnet ist, daß man zur Erzielung einer vorbestimmten Porenstruktur den Formkörper schichtenweise durch wiederholte Abfolge der Schritte
- Erzeugung einer entsprechend dem Porensystem bildartig-strukturierten Schicht aus der Masse
- Verfestigung der Schicht
aufbaut, wobei man die Bildstrukturen der einzelnen Schichten von entsprechenden Vorlagen überträgt.

Gegenstand der Erfindung ist insbesondere ein derartiges Verfahren, bei dem eine keramische Masse eingesetzt wird, wobei der Schritt der Verfestigung der Schicht durch Trocknung erfolgt und der erzeugte Formkörper in einem abschließenden Schritt gesintert wird.

Gegenstand der Erfindung ist weiterhin die Verwendung eines solchen Verfahrens zur Herstellung von Formkörpern mit dreidimensionalem interkonnektierenden Porensystem, insbesondere zur Herstellung von Implantatformkörpern mit dem Porensystem von natürlichen Knochen.

Dem erfindungsgemäßen Verfahren zur Herstellung poröser Formkörper mit vorbestimmter Porenstruktur liegt in allen seinen möglichen Ausführungsformen das Verfahrensprinzip zugrunde, daß man zunächst von einer Vorlage, also von einem als Muster dienenden porösen Körper, dessen Porenstruktur praktisch zu kopieren ist, die Bildinformation der Struktur schichtenweise aufnimmt, in einer für die jeweilige Fertigungstechnik geeigneten Form speichert und anschließend zur Steuerung des schichtweisen Aufbaus des Formkörpers verwendet. Bei als Vorlage dienenden realen Körpern kann die Aufnahme und Speicherung der Bildinformation etwa durch schichtenweises Abtragen des Körpers durch Schneiden, Sägen oder Schleifen und fotografische Aufnahme der jeweiligen Schichtoberflächen geschehen. Das Porensystembild kann aber auch künstlich konstruiert werden und in Form technischer Zeichnungen der Schichtbilder oder entsprechender bildlicher Darstellungen vorliegen.

Besonders vorteilhaft ist es, den Schritt der Aufnahme und Speicherung der schichtenweise Bildinformationen rechnergestützt und in digitalisierter Form vorzunehmen. So können beispielsweise die fotografischen Schichtaufnahmen oder die anderweitig erzeugten Schichtbilder mittels Laserscanner in einen Rechner, zweckmäßigerweise in ein CAD/CAM-System, überführt werden. Ebenfalls ist es möglich, Schichtstrukturbilder mittels Computertomographie (CT) aufzunehmen und in das Rechnersystem zu übertragen. Letzteres Verfahren bietet sich besonders für die Übertragung von Knochenstrukturen für die Fertigung von Implantatformkörpern an.

Die Übertragung der Bildinformationen zum schichtenweisen Aufbau des porösen Formkörpers aus einem geeigneten vorgewählten Material kann im einfachsten Fall in Form der Siebdrucktechnik erfolgen, wobei die fotografischen Schichtaufnahmen oder anderweitig erzeugten Schichtbilder in an sich bekannter Weise auf Siebdruckgewebe oder Siebdruckfolien übertragen werden. Das Material, das siebdruckfähig, also entsprechend plastisch verformbar und anschließend verfestigbar sein muß, wird durch das erste Sieb auf eine Unterlage gedruckt. Nach Verfestigung erfolgt das Aufdrucken durch das zweite Sieb und so fort, bis der Formkörper fertiggestellt ist.

Selbstverständlich läßt sich diese Verfahrensweise ebenfalls rechnergestützt realisieren, wobei beispielsweise die Schichtbildinformationen wiederum in einem CAD/CAM-System voliegen und die Erzeugung der Siebdruckfolien sowie die Bedienung einer entsprechenden Siebdruckanlage zum Aufbau des Formkörpers programmgesteuert erfolgt.

Spritzfähige Materialien können auch durch Spritztechnik verarbeitet werden, wobei die einzelnen Schichten durch dreidimensional steuerbare Düsen oder Düsensysteme aufgebaut werden. Gerade bei dieser Technik ist es besonders zweckmäßig, die Düsensteuerung rechnergestützt und programmgesteuert mit Hilfe eines CAD/CAM-Systems vorzunehmen. Die Kombination der digitalisierten Bildaufnahme, -steuerung und -verarbeitung mit programmgesteuerter Spritzdüsensteuerung läßt eine elegante vollautomatische Durchführung des erfindungsgemäßen Verfahrens zu. Es können hierzu herkömmliche Systeme und entsprechende Steuerprogramme zum Einsatz gelangen, die vom einschlägigen Fachmann ohne weiteres auf die spezifischen Belange des erfindungsgemäßen Verfahrens eingerichtet werden können.

Es versteht sich von selbst, daß sich dieses Verfahrensprinzip praktisch auf alle plastisch verformbaren und anschliessend verfestigbaren, somit also schichtweise verarbeitbaren Materialien anwenden läßt. Dies können bevorzugt plastifizierte oder verflüssigte keramische Massen sein, die zwischen den Schichtaufbauschritten durch Trocknung verfestigt und in einem abschließenden Schritt gesintert werden. Beispiele hierfür sind Kalziumphosphat-Keramiken, Oxidkeramiken und Glaskeramiken bzw. ihre entsprechenden Vorstufen. Bei der Dimensionierung des Schichtaufbaus und der Detailstrukturen ist der für keramische Massen typische Schwund einzukalkulieren.

Weiterhin können nichtkeramische mineralische Massen wie etwa hydraulische Zemente und ähnliche Materialien erfindungsgemäß verarbeitet werden.

Weitere erfindungsgemäß verarbeitbare Materialien können auf hochmolekularen, polymeren und/oder polymerisierbaren organischen Verbindungen basieren. Beispiele hierfür sind Wachse und Formulierungen auf Basis von lösungsmitteltrocknenden oder schmelzbaren Kunstharzen sowie Reaktivharzsystemen.

Schließlich können auch entsprechend verarbeitbare Kompositwerkstoffe aus zwei oder mehreren der vorgenannten Materialien, vorzugsweise aus keramischen Partikeln und organischen Polymermaterialien, zum Einsatz kommen.

Es versteht sich ebenfalls von selbst, daß das der Erfindung zugrundeliegende Verfahrensprinzip auch auf die Herstellung von Formkörpern praktisch beliebiger Dimensionierung und Formgebung sowie beliebiger Porenstruktur angewandt werden kann. Hierbei ist deren späterer Verwendungszweck keiner Beschränkung unterworfen. Bevorzugtes Anwendungsgebiet ist die Herstellung von Formkörpern mit dreidimensionalem interkonnektierenden Porensystem, insbesondere die Herstellung von Implantatformkörpern mit dem Porensystem von natürlichen Knochen.

Ein besonderer Vorteil des erfindungsgemäßen Verfahrens ist auch, daß bei dem künstlichen Aufbau poröser Formkörper anhand entsprechender Vorlagen im Bedarfsfalle gezielte Strukturveränderungen vorgesehen bzw. vorgenommen werden können. Solche Strukturveränderungen können beispielsweise im Sinne einer gezielten lokalen Verstärkung vorgenommen werden.

Implantatformkörper für den Knochenersatz können mit zusätzlichen Hohlräumen zu Aufnahme pharmazeutischer Wirkstoffe versehen werden. Hierdurch lassen sich beispielsweise Anti-biotika oder Wachstumsregulatoren pharmazeutisch definiert, lokal und mengenmäßig auf die Erfordernisse des Heilungsvorganges abgestimmt in dem Implantat plazieren. Eine nach bisheriger Technik über das Volumen im wesentlichen homogene Beladung des Implantats mit der zwangsläufig erforderlichen Überdosierung kann hiermit vermieden werden.

Die Durchführung des erfindungsgemäßen Verfahrens soll im folgenden beispielhaft anhand der Herstellung von Implantatformkörpern für den Knochenersatz näher erläutert werden.

Implantatformkörper können sowohl in Form eines Positiv- als auch eines Negativabbildes des zu ersetzenden Knochens bzw. Knochenbereiches hergestellt werden.

Die Positivform ist dann vorzuziehen, wenn das Implantat aus nichtresorbierbarer Kalziumphosphat-Keramik, wie insbesondere Hydroxylapatitkeramik, gefertigt werden soll. Die Einheilung des Implantats erfolgt dann im wesentlichen durch Einsprossung von körpereigenem Gewebe in die dem natürlichen Knochen weitestgehend entsprechenden Porenhohlräume des Implantatformkörpers. Die Herstellung des Implantatformkörpers kann gemäß dem oben allgemein beschriebenen Verfahrensprinzip beispielsweise nach den folgenden Verfahrensvarianten hergestellt werden:

### Ausführungsvariante 1

Natürlicher, nach bekannten Verfahren von organischen Anteilen befreiter Knochen wird schichtenweise abgetragen. Von jeder Schichtoberfläche wird eine fotografische Aufnahme gemacht. Diese Aufnahmen oder die Schichtoberflächen direkt werden mittels eines Laserscanners in ein CAD/CAM-System übertragen. In dem Rechnersystem erfolgt eine Ausrichtung und Duplizierung der Schichtaufnahmen. Diese Einzelschichten werden auf Positivfilm übertragen, mit dem entsprechende Drucksiebe erstellt werden.

Synthetisch gefälltes Hydroxylapatit wird mit einem gängigen organischen Verflüssiger zu einer verflüssigten keramischen Masse verarbeitet.

Diese Masse wird nun mit dem ersten Sieb zu einer Schicht auf eine Unterlage gedruckt. Es schließt eine Trocknung mit Heißluft an. Mit dem Sieb der zweiten Porenschichtaufnahme wird die zweite Lage auf die erste getrocknete Keramikschicht aufgetragen und wiederum getrocknet. So verfährt man bis alle Schichten gedruckt sind und ein keramischer Grünling vorliegt. Der erhaltene Grünling kann zusätzlich mit einem keramischen Schlicker gleicher Zusammensetzung zur Glättung der Schichtübergänge gespült werden.

Im Anschluß wird der Grünling zur Keramik gesintert, beispielsweise mit einem Sinterprogramm, das eine Aufheizrate von 50 K/Min. bis zu einer Temperatur von etwa 1300 °C und anschließend eine Haltezeit von 2 Stunden vorsieht. Nach der Abkühlung ist der Keramikformkörper fertig.

Je nach Erfordernis kann sich hieran noch eine mechanische Endformgebung anschließen, die mit üblichen Methoden (Schleifen, Drehen, Fräsen, Bohren usw.) jede gewünschte Implantatform liefert.

### Ausführungsvariante 2

Die im Rechnersystem gemäß Variante 1 gespeicherten Schichtbilder werden mittels einer CAD/CAM-gesteuerten Spritz- oder Auslaufdüse, die mit dem keramischen Schlicker beschickt wird, schichtenweise mit zwischengeschalteten Trocknungsphasen aufgetragen. Der so erhaltene Grünling wird analog zu Variante 1 weiter zur Keramik verarbeitet.

Ein Implantatformkörper in Form eines Negativabbildes der Knochenstruktur bietet sich dann an, wenn ein bioresorbierbarer Werkstoff eingesetzt werden soll. Die Einheilung eines solchen Implantatformkörpers erfolgt im Wesentlichen dadurch, daß der Werkstoff durch Bioresorptionsmechanismen langsam abgetragen wird und gleichzeitig ein Ersatz durch Einwachsen von körpereigenem Gewebe, insbesondere von neugebilderter mineralisierter Knochenmatrix, erfolgt.

Ein solcher Implantatformkörper kann gemäß dem allgemein beschriebenen Verfahrensprinzip beispielsweise nach den folgenden Verfahrensvarianten hergestellt werden:

### Ausführungsvariante 3

Gemaß den im Rechnersystem gespeicherten Schichtbildern wird nach dem Siebdruck- oder Spritzverfahren zunächst ein temporärer positiver Formkörper hergestellt. Als Material dient hier zweckmäßigerweise eine leicht verschmelzbare oder lösliche Masse, beispielsweise Wachs. Dieser Körper wird dann mit einem hochverflüssigten keramischen Schlicker auf Basis des bioresorbierbaren Tricalciumphosphat getränkt und getrocknet. Der Körper wird nun über den Schmelzpunkt des Wachses erhitzt, so daß das Wachs aus dem Körper herausläuft. Der erhaltene keramische Grünling mit dem Negativ des Poren- und Stegsystems eines natürlichen Knochens wird nun analog zu Variante 1 weiter zur Keramik verarbeitet. Man erhält einen bioresorbierbaren Implantatformkörper auf Basis von Tricalciumphosphat-Keramik.

### Ausführungsvariante 4

Gemäß den im Rechnersystem gespeicherten Schichtbildern wird mit einem keramischen Schlicker auf Basis von Tricalciumphosphat nach dem Siebdruck- oder Spritzverfahren ein Formkörper aufgebaut, der dem Negativ des Poren- und Stegsystems des natürlichen Knochens entspricht. Die Weiterverarbeitung des keramischen Grünlings zum keramischen Implantatformkörper erfolgt in bekannter Weise.

In dieser Variante können außer keramischen Massen auf Basis bioresorbierbarer Kalziumphosphate auch andere bioresorbierbare Materialien verarbeitet werden. Beispiele hierfür sind bioresorbierbare Polymere, beispielsweise Polylactide und Polyglycolide, sowie teilweise oder vollständig resorbierbare Kompositwerkstoffe auf Basis von Kalziumphosphatpartikeln und den vorgenannten Polymeren.

Nach dem erfindungsgemäßen Verfahren zur Herstellung poröser Formkörper mit vorbestimmter Porenstruktur können auch Formkörper für beliebige andere Anwendungszwecke hergestellt werden.

Ein Beispiel hierfür sind keramische Katalysatorträger für die Abgasreinigung von Automobilen. Nach gegenwärtiger Technik werden hierfür stranggepreßte Wabenformkörper eingesetzt, die entsprechend dieser Fertigungsmethode nur Längsporen aufweisen. Nach dem erfindungsgemäßen Verfahren hergestellte keramische Katalysatorträger mit dreidimensionaler interkonnektierender Porenstruktur können aufgrund wesentlich höherer innerer Oberfläche in ihrer Effektivität gesteigert bzw. in ihrer Baugröße reduziert werden.

Weitere Anwendungsgebiete sind poröse Körper für die Filtertechnik oder als statische Mischer für Gase bzw. Flüssigkeiten. Durch gezielten Aufbau der Porosität können hier die Strömungseigenschaften optimiert werden.

Ein weiteres Beispiel sind poröse Keramikformkörper, die als Sorbensmaterial für chromatographische Säulen dienen. Auch hier lassen sich durch vorgewählte Porenstruktur Strömungseigenschaften und Effektivität optimieren. Chromatographische Säulen mit Füllmaterialien in Form vorgefertigter, fester, poröser, keramischer Sorbensformkörper machen die aufwendigen Säulenpackungsprozeduren überflüssig und sind auch keiner Dichteänderung unterworfen. Ein keramischer Sorbenskörper kann auch problemlos regeneriert werden, etwa durch Ausbrennen bei je nach Basismaterial entsprechend hoher Temperatur.

## Patentansprüche

1. Verfahren zur Herstellung eines porösen Formkörpers durch Formgebung einer plastisch verformbaren und anschließend verfestigbaren Masse, dadurch gekennzeichnet, daß man zur Erzielung einer vorbestimmten Porenstruktur den Formkörper schichtenweise durch wiederholte Abfolge der Schritte
- Erzeugung einer entsprechend dem Porensystem bildartig strukturierten Schicht aus der Masse
- Verfestigung der Schicht
aufbaut, wobei man die Bildstrukturen der einzelnen Schichten von entsprechenden Vorlagen überträgt.

2. Verfahren nach Anspruch 1, dadurch gekennzeichnet, daß der Schichtaufbau mittels Siebdrucktechnik erfolgt.

3. Verfahren nach Anspruch 1, dadurch gekennzeichnet, daß der Schichtaufbau durch Spritztechnik erfolgt.

4. Verfahren nach einem der Ansprüche 1 bis 3, dadurch gekennzeichnet, daß die Übertragung der Bildstrukturen und der Schichtaufbau programmgesteuert erfolgt.

5. Verfahren nach Anspruch 4, dadurch gekennzeichnet, daß die Übertragung der Bildstrukturen in digitalisierter Form erfolgt.

6. Verfahren nach einem der Ansprüche 1 bis 5, dadurch gekennzeichnet, daß eine keramische Masse eingesetzt wird, wobei der Schritt der Verfestigung der Schicht durch Trocknung erfolgt und der erzeugte Formkörper in einem abschließenden Schritt gesintert wird.

7. Verfahren nach einem der Ansprüche 1 bis 5, dadurch gekennzeichnet, daß eine Masse auf Basis von polymerisierbaren organischen Verbindungen und/oder organischen Polymermaterialien eingesetzt wird.

8. Verfahren nach einem der Ansprüche 1 bis 5, dadurch gekennzeichnet, daß eine Masse auf Basis eines Kompositwerkstoffes aus keramischen Partikeln und organischen Polymermaterialien eingesetzt wird.

9. Verwendung des Verfahrens nach einem der Ansprüche 1 bis 8 zur Herstellung von Formkörpern mit dreidimensionalem interkonnektierenden Porensystem.

10. Verwendung nach Anspruch 9 zur Herstellung von Implantatformkörpern mit dem Porensystem von natürlichen Knochen.

11. Verwendung nach Anspruch 9 zur Herstellung von keramischen Katalysatorträgern.

12. Verwendung nach Anspruch 9 zur Herstellung von keramischen Filtermedien.

13. Verwendung nach Anspruch 9 zur Herstellung von keramischen Sorbensformkörpern für die Chromatographie.

## Claims

1. Process for producing a porous shaped body by shaping a composition capable of being plastically moulded and subsequently hardened, characterized in that to achieve a predetermined pore structure the shaped body is built up in layers by repetition in order of the steps
- production of a layer of the composition having a pattern-like structure corresponding to the pore system
- hardening of the layer,
where the pattern structures of the individual layers are transferred from corresponding masters.

2. Process according to Claim 1, characterized in that the build-up of layers is carried out by means of screen printing.

3. Process according to Claim 1, characterized in that the build-up of layers is carried out by spraying.

4. Process according to any of Claims 1 to 3, characterized in that the transfer of the pattern structures and the build-up of layers is carried out under the control of a program.

5. Process according to Claim 4, characterized in that the transfer of the pattern structures is carried out in digitized form.

6. Process according to any of Claims 1 to 5, characterized in that a ceramic composition is used, with the step of the hardening of the layer being carried out by drying and the shaped body produced being sintered in a subsequent step.

7. Process according to any of Claims 1 to 5, characterized in that a composition based on polymerizable organic compounds and/or organic polymer materials is used.

8. Process according to any of Claims 1 to 5, characterized in that a composition based on a composite material comprising ceramic particles and organic polymer materials is used.

9. Use of the process according to any of Claims 1 to 8 for producing shaped bodies having a three-dimensional, interconnecting pore system.

10. Use according to Claim 9 before producing shaped implants having the pore system of natural bone.

11. Use according to Claim 9 for producing ceramic catalyst supports.

12. Use according to Claim 9 for producing ceramic filter media.

13. Use according to Claim 9 for producing shaped ceramic sorbents for chromatography.

## Revendications

1. Procédé de fabrication d'un article poreux par façonnage d'une masse plastiquement déformable puis solidifiable, caractérisé en ce que pour obtenir une structure de pores prédéterminée on construit l'article couche par couche par succession répétée des étapes de
- production d'une couche structurée selon une image correspondant au système de pores à partir de la masse
- solidification de la couche,
dans lequel on transmet les structures d'images des diverses couches au moyen de modèles correspondants.

2. Procédé selon la revendication 1, caractérisé en ce qu'on réalise la construction des couches par une technique de sérigraphie.

3. Procédé selon la revendication 1, caractérisé en ce qu'on réalise la construction des couches par une technique de pulvérisation.

4. Procédé selon l'une des revendications 1 à 3, caractérisé en ce qu'on effectue la transmission des structures d'images et la construction des couches selon une commande par programme.

5. Procédé selon la revendication 4, caractérisé en ce que la transmission des structures d'images s'effectue sous forme numérisée.

6. Procédé selon l'une des revendications 1 à 5, caractérisé en ce qu'on utilise une masse de céramique, l'étape de solidification de la couche s'effectuant par séchage et l'article produit étant fritté dans une étape finale.

7. Procédé selon l'une des revendications 1 à 5, caractérisé en ce qu'on utilise une masse à base de composés organiques polymérisables et/ou de matières polymères organiques.

8. Procédé selon l'une des revendications 1 à 5, caractérisé en ce qu'on utilise une masse à base de matériau composite fait de particules céramiques et de matières polymères organiques.

9. Application du procédé selon l'une des revendications 1 à 8 à la préparation d'articles à système de pores interconnectés tridimensionnel.

10. Application selon la revendication 9 à la fabrication d'articles implantables ayant le système de pores des os naturels.

11. Application selon la revendication 9 à la fabrication de supports de catalyseurs céramiques.

12. Application selon la revendication 9 à la fabrication de milieux de filtration céramiques.

13. Application selon la revendication 9 à la fabrication d'articles de sorption pour la chromatographie.
